# EUROPEAN PATENT SPECIFICATION

(11) **EP 0 478 319 B1**
(45) Date of publication and mention of the grant of the patent: **02.04.1997**
(21) Application number: 91308770.6
(22) Date of filing: 26.09.1991
(51) Int. Cl.: C12Q 1/68

(54) **Gene detection method**
Verfahren zum Gennachweis
Méthode pour la détection de gènes

(30) Priority: 28.09.1990 JP 259011/90; 22.04.1991 JP 90879/91; 31.07.1991 JP 191868/91
(43) Date of publication of application: 01.04.1992
(73) Proprietor: KABUSHIKI KAISHA TOSHIBA, Kawasaki-shi, Kanagawa-ken 210 (JP)
(72) Inventor: Hashimoto, Koji, c/o Intellectual Property Div., Minato-ku, Toyko 105 (JP); Miwa, Keiko, c/o Intellectual Property Div., Minato-ku, Toyko 105 (JP); Ishimori, Yoshio,c/o Intellectual Property Div., Minato-ku, Toyko 105 (JP)
(74) Representative: Freed, Arthur Woolf

(56) References cited:
- EP-A- 0 149 339
- EP-A- 0 244 326
- EP-A- 0 245 206
- EP-A- 0 364 208
- WO-A-87/02066
- FR-A- 2 596 159
- GB-A- 2 217 007

## Description

This invention relates to a novel gene detection method to detect a certain gene specifically.

A genetic information stored in DNA is expressed as a protein or an enzyme through mRNA. By the effects of such protein or enzyme, various compounds necessary to maintain the vital actions are biosynthesized and metabolized. Thus a life is present as a dynamic equilibrium system of various substances controlled by genes.

There are 50 to 100 thousands of human genes. When some of them involve abnormality or change, such as defect or duplication, the characteristics, types and amounts of the proteins synthesized are changed, resulting in the poorly balanced biosystem, which may cause diseases. Thus, by detecting known pathogenic genes, the diseases may be identified or prevented. Such diagnosis based on the genes themselves has been developed as a results of the recently advancing technology of gene engineering, and is called as gene diagnosis.

When compared with conventional diagnostic methods, the gene diagnosis has characteristics as mentioned below.

Considering the mechanisms of gene expression, it can be presumed that changes in genes occur prior to almost all biochemical changes. Therefore, gene diagnosis by means of detecting the genetic change enables the diagnosis or prognosis prior to development of a disease which is one of phenotypes. Accordingly, the diagnosis and prognosis can be conducted before the development, in the latent period or at the earliest stage of the disease. This is the primary characteristic. As the secondary characteristic, gene diagnosis relating to the genetic diseases is independent from the organs or tissues to be analyzed since all genes in a living body are the same. This is particularly important in the diagnosis in fetus. Thus, this secondary characteristic enables the diagnosis simply by sampling amniotic fluid from a pregnant woman and analyzing the fetal cells suspending in the amniotic fluid.

Procedure of gene diagnosis conventionally employed is summarized as follows.

Genes are extracted from a samples and cleaved, if necessary, by appropriate restriction enzymes, and then subjected to electrophoresis and southern blotting. Then a nucleic acid probe (usually radiolabelled) having the base sequence complementary to the gene to be detected is hybridized to the blotted gene. Subsequently, the hybridized nucleic acid probe is detected by exposing an X-ray film to the radiation emitted from the labeled probe at lower temperature to confirm the presence of the gene.

The conventional detection method mentioned above involves the limitation of the place of diagnosis due to the use of radioisotopes and should be conducted with sufficient care of handling reagents. For the purpose of reducing such inconvenience, safe labeling agents substituting the radioisotopes are being developed and several detection methods, in which probes are utilized, such as avidin-biotin bond method or enzymatic or fluorescent method and the like have already been suggested. However, these methods can not achieve the sensitivity superior to that of the method using radioisotopes. They also involve the problems of the time period required to detecting the gene as long as 2 or 3 days as well as complicated procedure of determination.

On the other hand, quantification of a certain antigen or antibody present in a sample generally employs radioimmunoassay (RIA). However, RIA requires special instruments and authorised operators therefor capable of handling radioisotopes since this method also employs radioisotopes similarly as in the gene diagnosis methods mentioned above. In addition, waste disposal in this method should be done with particular care. As one of the other analytic methods, immunoelectrophoresis, which requires a long period for determination and has a poor sensitivity, can be suggested, although this method is not applicable in case of the samples'containing only trace amount of test substance.

EP-A-245 206 discloses a gene detection method in which a fluorescent intercalater and a waveguide tube such as an otpical fiber are employed. However, the use of an electrode as a carrier, or an intercalater having the properties including luminescence, phosphorescence, light absorption, etc., are not discussed in this document.

EP-A-149 339 discloses a method in which an electrochemically active mediator-coupled or an enzyme-coupled DNA probe is hybridized with a sample DNA in a solution in advance, and a signal resulting in the generation of the mediator itself, or the enzyme reaction is measured by an electrode. This method differs from the present invention in that a DNA probe is modified with a mediator or enzyme without using a double stranded nucleic acid recognizing substance. This document makes no mention of a double stranded nucleic acid recognizing substance.

EP-A-244 326 discloses a gene detection method in which a DNA probe is fixed on the surface of an electrode to make it hydridized with a sample DNA, and then the variance of the resistance or the electric capacity is measured.

The object of measurement by the electrode differs from that of the present invention. In addition, this document makes no mention of a double stranded nucleic acid recognizing substance.

GB-A-2 217 007 discloses a method which operates on basically the same principle of that of EP-A-149 339 except that the substance which modified the DNA probe is an electrochemically light-emitting substance. In this document, the electrode is used merely to apply a potential, but not used for detection of a signal. The light generated from the electrochemically light-emitting substance when a potential is applied is detected by a luminescence detector. A double standed nucleic acid recognizing substance is neither discussed nor suggested.

EP-A-135 159 disclosed a method of specifically detecting a target gene, in which a DNA probe is fixed to a carrier to make it hybridized with a sample DNA, and then reacted with a double stranded nucleic acid recognizing antibody. In this method, since an antibody itself is not capable of generating a signal, it is necessary to utilize an enzyme-substrate reaction or an immunological technique for detection. This document neither discusses nor suggests a double stranded nucleic acid recognizing substance having an electrochemical activity. Further, in this method, the carrier may be of any type as long as it can support a DNA probe, and may not be an electrode.

WO-A-8 604 244 discloses metal complexes which can be used as antitumour agents and which are intercalating agents, while EP-A-109 767 discloses a modified electrode wherein the surface of the electrode is covered with an "electron mediator". The substances which are used to coat the electrode are, specifically, redox/adsorption electron mediators. These mediators must react with the substances which they are detecting in an electrochemical manner, so that all of the examples of substances which can be detected by the electrodes of EP-A-109 767 are substances which, in nature, are involved in electron-transfer systems. Such substances include NADH, NADPH, cytochrome C₃, cytochrome C, haemoglobin and chlorophyll.

An objective of the present invention is to provide a method which is excellent in safety and convenience and is capable of detecting a certain gene at a high sensitivity in a reduced time period.

Accordingly, there is provided a gene detection method as claimed in any of Claims 1 to 11.

This invention can be more fully understood from the following detailed description when taken in conjunction with the accompanying drawings, in which:

Fig. 1 shows a diagram schematically representing an example of an automatic gene detection device suitable to be used in the present invention.

Fig. 2 shows a perspective view of another embodiment of the reaction bath and a gene sample purification device of the automatic gene detection device shown in Fig. 1.

Fig. 3 shows a perspective view of an example of the temperature controller in the automatic gene detection device shown in Fig. 1.

Fig. 4 shows a diagram schematically representing an example of the said automatic gene detection device utilizing electrochemiluminescene.

In the present invention, the term "double stranded nucleic acid recognizing substance" means a substance which recognizes and binds specifically to a double stranded nucleic acid. Such substances are, for example intercalating agents and biopolymers capable of recognizing double stranded nucleic acid.

The intercalating agents are characterized by their tendency to intercalate specifically to double stranded nucleic acid such as double stranded DNA. These intercalating agents have in their molecules a flat intercalating group such as phenyl group, which intercalates between the base pairs of the double stranded nucleic acid, whereby binding to the double stranded nucleic acid. The intercalating agents used in the present invention are electrochemically active or chemiluminescent and determination of these properties serves to detect the intercalating agents bound to a double stranded nucleic acid.

Electrochemically or optically active intercalating agents useful in the present invention are, but are not limited to, ethidium, ethidium bromide, acridine, aminoacridine, acridine orange, proflavin, ellipticine, actinomycin D, daunomycin, mitomycin C and the like. Other intercalating agents which are useful are those listed in Published Japanese Patent Application No. 62-282599.

In addition to the intercalating agents which are reversibly reacted themselves during oxidation-reduction reaction as listed above, the determination of electrochemical change using an electrode may employ a metal complex containing as a center metal a substance capable of undergoing electrically reversible oxidation-reduction reaction, namely, a metallo intercalater. Such metallo intercalaters include for example tris (phenanthroline) zinc salt, tris (phenanthroline) ruthenium salt, tris (phenanthroline) cobalt salt, di (phenthroline) zinc salt, di (phenanthroline) ruthenium salt, di (phenanthroline) cobalt salt, bipyridine cobalt salt, terpyridine platinum salt, phenanthroline platinum salt, tris (bipyridyl) zinc salt, tris (bipyridyl) ruthenium salt, tris (bipyridyl) cobalt salt, di (bipyridyl) zinc salt, di (bipyridyl) ruthenium salt, di (bipyridyl) cobalt salt and the like. Although the intercalating agents are not limited to those listed above, the complexes which or whose center metals have oxidation-reduction potentials not lower than or covered by that of nucleic acids are less preferable.

By using the intercalating agents capable of undergoing electrochemically reversible oxidation-reduction reaction, it is possible to determine the oxidation-reduction current repetitively. Accordingly, it is possible to conduct potential scanning several to several hundreds times and to sum up the values of the signals obtained, whereby enabling the amplification of the signals, resulting in a higher sensitivity of the detection.

When conducting the detection of the gene using an electrode, an intercalating agent exhibiting electrochemiluminescence may also be employed. Such intercalating agents are, but are not limited to, for example, luminol, lucigenin, pyrene, diphenylanthracene and rubrene. The electrochemiluminescene of the intercalating agents listed above may be enhanced by the enhancers such as luciferin derivatives such as firefly luciferin and dihydroluciferin, phenols such as phenyl phenol and chlorophenol as well as naphthols.

Optical signals generated by the electrochemiluminescence may directly be detected from the solution using, for example, photocounter. Alternatively, an optical fiber electrode produced by forming a transparent electrode at the tip of an optical fiber may also be used to detect the signal indirectly.

Since the electrode reaction or change in optical signal occur exclusively on the surface of the carrier, the detection can be conducted quite easily without removing unreacted probe or unreacted intercalating agent

In the present invention, reaction of nucleic acid probe and the single stranded gene sample is generally conducted in a solution. Such reaction may be conducted in the presence of the intercalating agents listed above or the intercalating agents may be added after completion of the reaction.

As mentioned above, since most of the intercalating agents have themselves the optical activity or can exhibit the electrode response, direct determination is possible by means of optical or electrochemical procedure. When these intercalating agents are further bound with the substances which generate signals capable of being detected directly or indirectly, higher detection sensitivity can be obtained by determining the signals combined with the signals from the intercalating agents.

These substances which generate signals capable of being detected directly or indirectly include, for example, haptens such as biotin, trinitrobenzene sulfonic acid and dinitrobenzene sulfonic acid, fluorescent substances such as fluorescein isothiocyanate (FITC), phycocyanin and rhodamine, luminescent substances such as luminol, lucigenin and acridium ester derivatives as well as electrode active substances such as ferrocene and viologen. When using the substance, from which the signal can not directly being detected, such as the haptens listed above, enzyme-labelled anti-hapten antibodies such as enzyme-labelled avidin are used to determine the optical parameters such as absorbance, fluorescene, luminescene, quenching, circular dichroism and fluorescene polarization or, electrode activity is determined, whereby indirectly detecting the gene.

Although one molecule of these substances are usually bound to one molecule of a intercalating agent, several molecules of these substance may be bound to one molecule of the intercalating agent, whereby enhancing the sensitivity.

The amount of the double stranded nucleic acid recognizing substance to be added is not particularly specified, although the amounts sufficient to bind all double strands formed are preferable in view of the efficiency. When added in an excess amount, the double stranded nucleic acid recognizing substance remaining unreacted are washed off prior to the determination.

When the amount of the double stranded nucleic acid recognizing substance added is small and the concentration of it is low, only small amount of the unreacted recognizing substance remains in the system after the recognizing substance has bound to the double stranded nucleic acid formed. Thus, the double stranded nucleic acid recognizing substance is relatively concentrated on the carrier. In such state, the gene can be detected without washing off the sample DNA which has not reacted with the nucleic acid probe or free double stranded nucleic acid recognizing substance which has not bound to the double stranded nucleic acid formed, whereby enabling the continuous reactions from hybridization through detection of the gene intended in a single system.

In the present invention, by varying the nucleic acid probe employed, various types of genes can be detected. Nucleic acid probes useful are such probes that have the base sequences complementary to entire or a part of base sequence of any of microorganisms contained in foods, plant viruses or viroids, pathogenic microorganisms or viruses infecting fishes, pathogenic microorganisms or viruses infecting human and causing infectious diseases, genes causing genetic diseases, activated proto-oncogenes and minisatellite sequence.

When using as a nucleic acid probe a probe having the base sequence complementary to entire or a part of the base sequence of a microorganism contained in a food, the microorganism contained in the food can directly be detected, thus enabling the food sanitary inspection. Such microorganisms contained in foods are, for example, pathogenic Escherichia coil, Staphylococcus as well as Salmonella.

When using as a nucleic acid probe a probe having the base sequence complementary to entire or a part of the base sequence of a plant virus or viroid, the plant virus or viroid with which plants are infected can be detected, thus enabling the infection diagnosis in agricultural fields. Examples of such plant viruses or viroids include tabacco mosaic virus and cauliflower mosaic virus.

When using as a nucleic acid probe a probe having the base sequence complementary to entire or a part of the base sequence of a pathogenic microorganism or virus infecting fishes, the pathogenic microorganism or virus with which fishes are infected can be detected, thus enabling the infection diagnosis in fishery field. Examples of such pathogenic microorganisms or viruses infecting fishes are pathogenic vibrio.

When using as a nucleic acid probe a probe having the base sequence complementary to entire or a part of the base sequence of a pathogenic microorganism or virus infecting human and causing infectious diseases, it is possible to conduct the infection diagnosis. Such pathogenic microorganisms infecting human and causing infectious diseases are, for example, pathogenic Streptococcus, Mycoplasma, Clostridium, Chlamydia, Salmonella, herpes simplex and cytomegalovirus.

When using as a nucleic acid probe a probe having the base sequence complementary to entire or a part of the base sequence of a gene causing a genetic disease, direct assay of the genetic disease is possible. Such genes causing genetic diseases are, for example, the genes causing adenosine deaminase deficiency and drepanocythemia.

When using as a nucleic acid probe a probe having the base sequence complementary to entire or a part of the base sequence of an activated proto-oncogene, it is possible to conduct the cancer diagnosis. Such activated proto-oncogenes are, for example, oncogenes listed in "Oncogene data book" (M. Shibuya, Pub. by Shujun-Sha).

When using as a nucleic acid probe a probe having the base sequence complementary to entire or a part of the base sequence of a minisatellite sequence, DNA finger print method useful for genetic studies, individual identification and parentage test can be conducted. Such minisatellite sequences are, for example, Myo sequence, Alu sequence, Per-6 sequence and Per sequence.

Although the length of a nucleic acid probe used in the present invention is not particularly specified and single stranded nucleic acids consisting of several to hundreds monomers may be employed, the lengths consisting of more than ten and less than a hundred monomers are preferable in order to increase S/N ratio and to obtain higher sensitivity in view of the fact describe below.

As already mentioned, a double stranded nucleic acid recognizing substance is such a substance that recognizes and binds specifically to a double stranded nucleic acid. However, the double stranded nucleic acid recognizing substance may bind to a single stranded nucleic acid in rare cases. Thus, it may bind to an unreacted nucleic acid probe immobilized on a carrier. When it binds in such a manner, S/N ratio is reduced, resulting in a poor detection accuracy. Accordingly, it is preferable to minimize the length of the nucleic acid probe as far as the detection of the intended gene is possible.

In the present invention, a nucleic acid probe is immobilized onto an electrode.

The electrodes employed in the present invention may be, but are not limited to, carbon electrodes such as graphite, glassy carbon, pyrolytic graphite, carbon paste and carbon fiber, noble metal electrodes such as platinum, platinum black, gold, palladium and rhodium, oxide electrodes such as titanium oxide, tin oxide, manganese oxide and lead oxide, semiconductor electrodes such as Si, Ge, ZnO, CdS, TiO₂ and GaAs, as well as titanium. These electrodes may also be covered with conductive polymers to enhance the stability of the electrodes immobilized with probes. Monomolecular films may also be employed to cover the electrodes.

The nucleic acid probes may be immobilized onto the surfaces of the electrodes by means of covalent bond, ionic bond and physical adsorption.

The examples of the procedure for the immobilization by means of covalent bond are the method in which the surface of the carrier is activated and then the nucleic acid probe is immobilized directly or indirectly through crosslinking agent and the method in which an active functional group is introduced into the nucleic acid probe to be immobilized onto the carrier followed by direct or indirect immobilization. The activation of the carrier surface may be conducted by electrolytic oxidation in the presence of oxidizing agent, or by air oxidation or reagent oxidation, as well as by covering with a film. Useful crosslinking agents may be, but are not limited to, silane couplers such as cyanogen bromide and gamma-aminopropyl triethoxy silane, carbodiimide and thionyl chloride and the like. Useful functional groups to be introduced to the nucleic acid probe may be, but are not limited to, amino group, carboxyl group, hydroxyl group, carbonyl group, phosphate group, aldehyde group and mercapto group. Other highly reactive functional groups may also be employed.

Once the surface of the carrier is oxidized for activation, an oxidized layer is formed on the surface. The nucleic acid probe binds to the substrate via this oxidized layer. By forming a thinner oxidized layer, S/N ration of detecting the gene is improved. The thickness of the oxidized layer is preferably not more than 500 A, more preferably not more than 100 A.

Introduction of the functional group into the terminal of the nucleic acid can be conducted by means of enzymatic reaction or by using DNA synthesizer. The enzymes useful in the enzymatic reaction may be, for example, terminal deoxynucleotidyl transferase, poly A polymerase, polynucleotide kinase, DNA polymerase, polynucleotide adenylyltransferase and RNA ligase. Polymerase chain reaction method (PCR method), nick translation method and random primer method may also be employed to introduce the functional group.

The functional group may be introduced to any part of the nucleic acid, such as 3' or 5' terminal, as well as a site randomly selected.

The nucleic acid probe with the functional group introduced may be immobilized as it is onto the carrier by immobilization reaction. However, the amino acid originally comprised in the nucleic acid may sometimes serve as a functional group instead of the functional group introduced, since the nucleic acid probe is a single strand. Thus the amino acid originally comprised in the nucleic acid is used for immobilization of the probe to the carrier, which affects the sensitivity.

The immobilization via the amino acid originally comprised in the nucleic acid probe may be prevented by, for example, the method as follows. First, the nucleic acid probe to which the functional group has been introduced is annealed with the DNA chain having the base sequence complementary to the probe to obtain a double strand. Then the functional group introduced is used to immobilize the double stranded nucleic acid to the carrier. Subsequently, thermal denaturation is conducted to form a single strand, whereby removing the DNA chain to which the functional group has not been introduced. The temperature during the thermal denaturation may usually be maintained from 90 to 98°C.

When the carrier to be used for immobilization of the nucleic acid probe is an electrode, the nucleic acid probe can be immobilized easily at a higher efficiency by utilizing physical adsorption. The physical adsorption of the nucleic acid probe onto the electrode surface can be conducted, for example, as follows. First, the electrode surface is washed with distilled water and alcohol using ultrasonic cleaner. Then the electrode is placed in the phosphate buffer (pH 7.0) containing the nucleic acid probe to adsorb the probe onto the surface of the substrate. During this procedure, potential of 0 to +1.0 V, preferably 0 to +0.1 V is applied to the electrode to accelerate the adsorption. Then the electrode having the nucleic acid adsorbed is placed in the solution containing nucleotides (ATP, CTP, GTP, TTP, dATP, dCTP, dGTP, dTTP and the like), and the electrode surface is coated with the nucleotides preferably while applying the potential of 0 to 1.0V. By this treatment, non-specific adsorption of the sample nucleic acid or double stranded nucleic acid recognizing recognizing substance to the electrode surface can be prevented. Non-specific adsorption may also be prevented by using surfactants, fatty acids or fats.

The nucleic acid probe may also be immobilized to the carrier using a embedding agent used in the embedding method known as a procedure to immobilize the enzyme. The embedding agents useful in the present invention may be, but are not limited to, polyvinylchloride and polyacrylamide.

The nucleic acid probe may also be immobilized onto the electrode surface via a film. Such films may be, for example, conductive polymers such as polyacethylene, polypyrrole, polythiophene and polyaniline as well as polyethylene, polypropylene, poly (vinylchloride), poly (vinyl alcohol), poly (methylmethacrylate), poly (vinyl-idene fluoride), cellulose and lipid membrane. Monomolecular layer such as LB membrane and a multiple layer consisting of two or more monomolecular layers may also be employed.

Immobilization of the nucleic acid to the film or the membrane may be conducted in the manner similar to that used for the immobilization onto the surface of the carrier.

When the nucleic acid probe is immobilized to the film or the membrane by means of covalent bond, a functional group may be introduced to the film or the membrane instead of the nucleic acid probe. The functional groups suitable to be introduced into the film or the membrane may be same to those introduced into the nucleic acid probe. By introducing the functional group into the film or the membrane followed by reacting the nucleic acid probe to effect the immobilization, the probe can be immobilized at a higher density and more stably immobilized carrier with nucleic acid probe can be obtained when compared to the immobilization by introducing the functional group into the nucleic acid probe.

When the carrier to which the nucleic acid probe is immobilized via the film or the membrane is an electrode, the probe and the sample are hybridized as mentioned above while determining the membrane potentials before and after hybridization, whereby detecting the presence of the intended gene.

A carrier having the nucleic acid probe immobilized, when used as it is, tends to exhibit non-specific physical adsorption caused by a sample nucleic acid and a double stranded nucleic acid recognizing substance. Such non-specific adsorption may cause a reduced sensitivity. It can be suppressed by covering the carrier surface with the nucleic acid by means of physical adsorption or chemical bond after immobilization of the nucleic acid probe.

For this purpose, the nucleic acids to be used to cover the surface of the carrier are, for example, nucleo-sides such as adenosine, thymidine, guanosine and cytidine, nucleotides such as uridylic acid, cytidylic acid, adenylic acid and guanylic acid, synthetic oligonucleotide as well as naturally derived DNA such as salmon sperm DNA.

While the length and the base sequence of nucleic acid used to cover the surface of the carrier are not limited specifically as far as they do not cause the reaction with the nucleic acid probe immobilized on the surface of the carrier, a single stranded or double stranded nucleic acid of 1 to 100 bp is preferable.

Non-specific adsorption can also be suppressed by coating the carrier with the substances such as surfactants, fatty acids and fats. An example of such substance is stearylamine.

In the gene detection method according to the present invention, the methods of immobilizing the nucleic acid probe to the carrier is not limited to those mentioned above and other method generally utilized to immobilizing biopolymers such as proteins to solid phases may widely be employed.

The amount of the nucleic acid probe to be immobilized to the carrier is not particularly limited, although a higher density of the nucleic acid probe immobilized results in a higher sensitivity of the detection, namely, a higher S/N ratio. The density of the nucleic acid probe immobilized is generally of the order of amol/cm² or higher.

The nucleic acid probe immobilized to an electrode can be quantified by determining an oxidation-reduction curren or an optical signal of the nucleic acid or by determining an oxidation-reduction current or an optical signal of an electrochemically or optically active substance which binds specifically to the single stranded nucleic acid. Thus, when the carrier is an electrode, then the oxidation reduction current from the nucleic acid or the intercalating agent is determined using a measurement system consisting of, for example, potentiostat, function generator, recorder and computer to quantify the nucleic acid immobilized. By using these methods, it is possible to quantify the nucleic acid immobilized on the surface of the carrier more easily in a shorter period at a higher sensitivity when compared with the conventional methods which are quite complicated since the nucleic acid itself has no activity. The oxidation-reduction current from the nucleic acid may be the oxidation-reduction current from adenine, thymine, guanine or cytosine.

By imparting the function of an oscillator or rotator to the carrier immobilized with the nucleic acid probe, the flow near the surface of the carrier can relatively be increased, whereby facilitating the htbridization reaction and inhibiting the non-specific reaction, providing higher efficiency of the gene detection. The function of oscillator can be imparted to the carrier by utilizing mechanical oscillation, ultrasonic wave or electric or magnetic action.

As test samples, blood such as peripheral venous blood, leukocyte, serum, urine, feces, semen, saliva, cultured cell, tissue cells such as cells from various organs and other materials containing nucleic acids may be employed.

Although the extraction of nucleic acids from a test sample is conducted according to the conventional method, the double stranded nucleic acid recognizing substance mentioned above can be used for extraction and purification following the procedure described below.

First, the double stranded nucleic acid recognizing substance is immobilized to an appropriate support, which is then mixed with a test sample. The cells contained in the test sample is ruptured to release the nucleic acid, which is then bound to the double stranded nucleic acid recognizing substance. Subsequently, the support is separated from the test sample and the nucleic acid bound to the double stranded nucleic acid recognizing substance is separated from the support.

The supports useful herein may be, but are not limited to, polymer supports such as latex, polyethylene, polystyrene, and polypropylene, carbon materials such as activated carbon, metal particulates, ceramic materials, and magnetic materials such as magnetites, samarium-cobalt and ferrite. The forms of the supports are not particularly limited, but a particle whose size is within the range from 0.1 to 1000 µm, particularly from 1 to 100 µm, is preferable.

The cells contained in the test samples may be ruptures according to the standard method. For example, the support is agitated by means of the external force such as shaking or applying ultrasonic wave. Nucleic acid eluate may also be employed to release the nucleic acid from the cells. Such nucleic acid eluates are, for example, the solutions containing surfactants such as SDS, Triton-X and Tween-20 (Triton and Tween are registered trade marks), and the solutions containing saponin, EDTA or proteases. When these solutions are used to release the nucleic acid, incubation at a temperature not lower than 37°C may serve to facilitate the reaction.

After binding the nucleic acid to the double stranded nucleic acid recognizing substance immobilized to the support, the support is separated from the test sample by an appropriate means. The support which has been separated is first washed with a washing fluid (low salt concentration) to remove the unnecessary components, and then with a nucleic acid eluate (high salt concentration) to release the nucleic acid into the solution. When an intercalating agent is used as the double stranded nucleic acid recognizing agent, a non-polar organic solvent is used as the nucleic acid eluate.

When a magnetic particle is used to the support, agitation and separation of the support can conveniently be conducted by using external magnetic action which enables easier and rapid procedure.

When the content of the intended gene is quite small, the detection may also be conducted after amplification of the gene according to a known method. Representative gene amplification methods are methods utilizing enzymes, such as polymerase chain reaction (PCR) method. The enzymes useful in the gene amplification method may be, for example, DNA-dependent DNA polymerazes such as DNA polymerase and Taq polymerase, DNA-dependent RNA polymerazes such as RNA polymerase I, RNA-dependent RNA polymerazes such as Q beta replicase. Among methods using these enzymes, PCR method using Taq polymerase is a quite convenient method wherein the amplification can be repeated continuously only by controlling the temperature.

The samples thus obtained (course extract of nucleic acid, or purified solution of nucleic acid) are thermally denaturated at a temperature form 90 to 98°C, preferably not lower than 95°C to prepare single strands. Then an electrode immobilized with nucleic acid probe or an optical fiber immobilized with nucleic acid probe is placed in the solution of the single stranded nucleic acid and hybridized at a temperature from 37 to 72°C. The optimum temperature for hybridization varies depending on the factors such as the base sequence and the length of the probe employed.

In such cases, the reaction rate is generally less satisfactory than in the case of the reaction in solution, since the hybridization reaction proceeds in a solid phase. However, this problem can be eliminated when using an electrode immobilized with the nucleic acid probe by applying a potential to the electrode surface before and/or during the hybridization raction to accelerate the reaction. The potential to be applied is preferably a positive potential, or a positive and negative potentials are alternatively applied. Such potential is applied continuously or intermittently as pulse. Preferably the potential applied is within the range from 0 to ±2.0 V.

Upon hybridization, unreacted nucleic acid may adsorb non-specifically to the electrode surface in addition to the intended gene bound to the nucleic acid probe. Such non-specific adsorption may reduce the S/N ratio of the gene detection. Usually the nucleic acid is negatively charged. Therefore the nucleic acid non-specifically adsorbed can be removed by applying the negative potential to the electrode. For this purpose, the potential to be applied is within the range from 0 to 2.0 V, preferably 0 to 1.5 V.

The double stranded nucleic acid recognizing substance may be added to the test sample either before or after the hybridization. Alternatively, a solution of the double stranded nucleic acid recognizing substance may previously be prepared, in which the electrode or optical fiber immobilized with the nucleic acid probe may be placed after hybridization. Since most of the double stranded nucleic acid recognizing substances are positively charged, non-specific adsorption of the recognizing substance to the carrier can be suppressed by applying positive potential in the cases where the carrier is an electrode.

Since the electrode reaction proceeds only near the surface of the electrode, a response of the intercalating agent bound to the double stranded nucleic acid to the electrode can be obtained exlcusively upon occurrence of hybridization. When using an electrode immobilized with a nucleic acid probe, a measurement system consisting of potentiostat, function generator and recorder may be used. Once the potential is set approximately to the oxidation-reduction potential of the intercalating agent, the potential is scanned. During the scanning, the oxidation-reduction current is determined to quantify the gene to be detected. This electrochemical determination may be done in a test solution or in other electrolyte solutions. It may also be done in hydrophilic or hydrophobic solvents.

The devices involving a nucleic acid probe immobilized to an electrode immobilized with the nucleic acid probe as mentioned above, are useful as gene detecting sensors. In order to use such devices as gene detecting sensors repeatedly, it is required to dissociate the sample which had been hybridized with the probe immobilized. Dissociation of the sample from the probe may be conducted by treatments with heat, bases, acids, surfactants or ultrasonic wave. The heat treatment can be done by heating the double stranded nucleic acid formed at 98°C for 5 minutes to denature the double stranded nucleic acid followed by rapid cooling. Alkaline treatment employs a buffer solution of pH 8.5 or higher or strong base solutions, while acid treatment employs a buffer solution of pH 4.5 or lower or strong acid solution. The surfactants used in the treatment with surfactants may be, but are not limited to, ionic or neutral surfactants such as SDS, Triton-X and Tween 20. For this purpose, the concentration of the surfactant is preferably 0.1% or higher. Ultrasonic treatment can be conducted by treating the sample for several seconds to several minutes with the ultrasonic wave having the frequency from 10 to 100 kHz.

The gene detection methods of the present invention are further described in the examples below.

Example 1: Gene detection using electrode immobilized with nucleic acid probe
a. Immobilization of nucleic acid probe to Pt electrode surface
   Pt electrode was treated at a high temperature to air-oxidize the surface of the electrode. After activating the surface of the oxide layer by cyanogen bromide (CNBr), immobilization was conducted by immersing the electrode in the solution of thermally denatured single stranded nucleic acid probe (v-myc).
b. Gene detection using electrode immobilized with nucleic acid probe

As a test sample, pVM623 obtained by inserting v-myc fragment into pst I site of pUC 119 was employed. Linear pVM 623 was obtained by digesting with Hind III and then was subjected to the thermal denaturation at 98°C. The electrode immobilized with the nucleic acid probe was placed in the test sample, which is incubated for 15 minutes at 70°C to anneal. During this procedure, acridine orange, an intercalating agent which has specificity to the double stranded DNA and is electrochemically active, was added.

After annealing, the electrode reaction was effected while determining the oxidation-reduction current produced to quantify v-myc contained in the test sample. As a result, v-myc could be detected in pg (picogram) order.

### Example 2: Gene detection using electrode immobilized with nucleic acid probe and using metallo intercalator as an intercalating agent

As a test sample, pVM 623 obtained by inserting v-myc fragment into Pst I site of pUC 119 was employed. Linear pVM 623 was obtained by digesting with Hind III and then was subjected to the thermal denaturation at 98°C. The electrode immobilized with the nucleic acid probe was placed in the test sample, which was incubated for 15 minutes at 70°C to anneal. During this procedure, tris (1,10-phenanthroline) cobalt (III), an intercalating agent which has specificity to the double stranded DNA and is electrochemically active, was added.

After annealing, cyclic voltammetry was conducted and values of the oxidation-reduction current obtained by sweeping 30 times were summed up. As a result, v-myc could be detected in pg order.

### Reference Example 1: Quantification of nucleic acid probe on BPPG electrode

a. Introduction of amino group into nucleic acid probe
   A DNA labeling kit, Chemiprobe (ORGENICS Ltd.) was used to label carcinogenic gene v-myc (1.5 Kb), into which (6-aminohexyl) dATP was then introduced at 3' terminal using terminal deoxynucleotidyl transferase.
b. Immobilization of nucleic acid probe to electrode surface
   As an electrode to which the nucleic acid is to be immobilized, basal plain pyrolytic graphite (BPPG) was employed. This electrode was subjected to electrolysis at 2.2 V in a solution containing 10% nitric acid and 2.5% potassium chromate to oxidize the surface. The electrode whose surface has been oxidized was then placed in a refluxed solution of 10% gamma-aminopropyl triethoxy silane in toluene at 120°C for 30 minutes to effect silane treatment. After the electrode was washed with methanol, it was reacted in 1% glutaraldehyde solution for 30 minutes, and was washed again. The electrode was reacted in a solution containing 1 µg/mℓ v-myc having amino group introduced at a room temperature for 30 minutes, whereby producing an electrode immobilized with the nucleic acid probe.
c. Quantification of nucleic acid probe immobilized to electrode surface
   Using the electrode immobilized with the nucleic acid probe thus obtained, cyclic voltammetry was conducted in 1/15M phosphate buffer solution (pH 7.0). As a result, oxidation current of 1 µA generated from adenine was measured by the electrode which had been subjected to 10 seconds oxidation, while oxidation current of 2 µA measured by the electrode which had been subjected to 60 seconds oxidation. From the determinatio using Chemiprobe kit, the amounts of nucleic acid immobilized to the surfaces of these electrodes were about 0.1 µmol/cm² and 0.2 µmol/cm², respectively. Such findings indicated that there may be correlation between the oxidation currents from the nucleic acids and the amounts of nucleic acid probe immobilized and that the nucleic acid probe immobilized may be quantified based on the electrode reaction of the nucleic acid.

### Example 3: Acceleration of hybridization of nucleic acid sample with nucleic acid probe immobilized to electrode

As a test sample, pVM 623 obtained by inserting v-myc fragment into Pst I site of pUC 119 was employed. Linear pVM 623 was obtained by digesting with Hind III and then was subjected to the thermal denaturation at 98°C. Then the BPPG electrode immobilized with the nucleic acid probe was placed in the test sample, which was incubated for 15 minutes at 70°C to effect annealing. During this procedure, 0.1 V (vs SCE) of potential was applied to the electrode.

Then acridine orange, which has specificity to the double stranded DNA and is electrochemically active, was added to effect the electrode reaction, during which the oxidation-reduction current generated was determined to quantify v-myc contained in the test sample. As a result, v-myc could be detected in pg order. The time required for hybridization which had conventionally been about 30 minutes was reduced to about 10 minutes.

### Example 4: Regeneration of electrode immobilized with nucleic acid probe

a. Immobilization of nucleic acid probe to Pt electrode surface
   Pt electrode was treated at a high temperature to air-oxidize the surface of the electrode. After activating the surface of the oxide layer by cyanogen bromide (CNBr), immobilization was conducted by immersing the electrode in the solution containing thermally denatured single stranded nucleic acid probe (v-myc).
b. Gene detection using electrode immobilized with nucleic acid probe
   As a test sample, pVM 623 obtained by inserting v-myc fragment into Pst I site of pUC 119 was employed. Linear pVM 623 was obtained by digesting with Hind III and then was subjected to the thermal denaturation at 98°C. The electrode immobilized with the nucleic acid probe was placed in the test sample, which was incubated for 15 minutes at 70°C to anneal. During this procedure, acridine orange, an intercalating agent which has specificity to the double stranded DNA and is electrochemically active, was added.
   After annealing, the electrode reaction was effected while determining the oxidation-reduction current produced to quantify v-myc contained in the test sample. As a result, v-myc could be detected in pg order.
c. Regeneration of electrode immobilized with nucleic acid probe
   By heating at 98°C for five minutes the electrode immobilized with the nucleic acid probe which had once been used for determination, the sample pVM 623 was dissociated from the surface of the electrode immobilized with the nucleic acid probe. The electrode thus regenerated could be reused for the gene detection repeatedly at least 5 times thereafter.

### Example 5: Gene detection using intercalating agent to which substance generating signals which can be detected directly or indirectly is bound.

a. Preparation of electrode immobilized with nucleic acid probe
   (6-aminohexyl) dATP was introduced at 3' terminal into synthetic oligonucleotide probe (20 mer) against to a carcinogenic gene v-myc using terminal deoxynucleotidyl transferase.
   On the other hand, a basal plain pyrolytic graphite (BPPG) electrode was subjected to electrolysis at 2.2 V in a solution containing 10% nitric acid and 2.5% potassium chromate to oxidize the surface of BPPG electrode. The electrode was then placed in a refluxed solution of 10% gamma-APTES in aniline at 120°C for 30 minutes to effect silane treatment. After the electrode was washed with methanol, it was reacted in 1% glutaraldehyde solution for 30 minutes and washed again.
   This BPPG electrode was reacted in a solution containing 1 µg/mℓ synthetic oligonucleotide probe, whcih has specificity to v-myc, having amino group introduced at a room temperature for 30 minutes, whereby producing an electrode immobilized with the nucleic acid probe.
b. Gene detection using electrode immobilized with nucleic acid probe

As a test sample, pVM 623 obtained by inserting v-myc fragment into Pst I site of pUC 119 was employed. Linear pVM 623 was obtained by digesting with Hind III and then was subjected to the thermal denaturation at 98°C. BPPG electrode immoblized with the nucleic acid probe was place in the test sample, which was incubated at 70°C to anneal. Subsequently, acridine orange bound to ferrocene was added in such an amount that final concentration of 1 µM was obtained. After washing, it was subjected directly to the electrode reaction, during which the oxidation-reduction current was determined to quantify v-myc contained in the sample.

When the sample did not contained the gene intended, then the oxidation-reduction current from ferrocene was not detected. However, in the case of the sample containing the gene intended, the oxidation-reduction current could be detected, and ultimately v-myc can be detected in the order of pg. Since no B/F separation was required, the detection was completed within 30 minutes.

### Example 6: Gene detection using intercalating agent to which substance generating signals which can be detected directly or indirectly is bound

First, the electrode immobilized with the nucleic acid probe was prepared in the manner similar as in Example 10, and then it was immersed in a solution of nucleotides (dATP, dCTP, dGTP and dTTP) for the purpose of preventing non-specific adsorption of sample DNA.

As a test sample, pVM 623 obtained by inserting v-myc fragment into Pst I site of pUC 119 was employed. Llnear pVM 623 was obtained by digesting with Hind III and then was subjected to the thermal denaturation at 98°C. Then BPPG electrode immobilized with the nucleic acid probe was placed in the test sample, which was incubated at 70°C to anneal. During this procedure, 0.1 V (vs SCE) of potential was applied to the electrode.

After reaction, an intercalating agent which has specificity to the double stranded DNA and is electrochemically active, namely tris (phenanthroline) cobalt salt was added. After tris (phenanthroline) cobalt salt was bound to the double stranded nucleic acid, negative charge was applied to the electrode to remove the substances binding non-specifically. Subsequently, the oxidation-reduction current of the intercalating agent was determined to quantify v-myc contained in the test sample. As a result, v-myc could be detected in pg order.

### Reference Example 2: Immobilization to electrode surface via amino group introduced into nucleic acid probe

a. Preparation of nucleic acid probe into which amino group is introduced
   2 primers (20 mer) used for amplification of about 1.0 Kb fragment of carcinogenic gene v-myc were synthesized using DNA synthesizer (Applied biosystem, PCR-MATE EP). An amino group was further introduced to one of the primer at 5' terminal using Aminolink 2 (Applied biosystem).
   These two synthetic primers were admixed each in the concentration of 100 µg/mℓ and subjected to the treatment at 95°C for 5 minutes followed by 37°C for 30 minutes to anneal to obtain a double stranded nucleic acid probe.
b. Immobilization of nucleic acid probe to electrode surface
   As an electrode to which the nucleic acid is to be immobilized, basal plain pyrolytic graphite (BPPG) was employed. This electrode was subjected to electrolysis at 2.2 V in a solution containing 10% nitric acid and 2.5% potassium chromate to oxidize the surface. The electrode whose surface had been oxidized was then placed in a refluxed solution of 10% gamma-APTES in aniline at 120°C for 30 minutes to effect silane treatment. After the silane treated electrode was washed with methanol, it was reacted in 1% glutaraldehyde solution for 30 minutes, and then washed again.
   This BPPG electrode was reacted in a solution containing 100 µg/mℓ of double stranded nucleic acid probe prepared in step a at a room temperature for 30 minutes, whereby immobilizing the double stranded nucleic acid on the surface of the electrode. Then the electrode was further subjected to thermal denaturation at 95°C for 5 minutes to remove the DNA chain without the functional group introduced, whereby producing an electrode immobilized with the nucleic acid probe.

### Example 7: Gene detection using electrode immobilized with nucleic acid probe via lipid membrance

a. Preparation of electrode immobilized with nucleicacid probe
   Lipid membrane was formed using phosphatidylethanolamine on the surface of basal plain pyrolytic graphite (BPPG) electrode. On the other hand, (6-aminohexyl) dATP was introduced at 3' terminal into carcinogenic gene v-myc (1.5 Kb) using terminal deoxynucleotidyl transferase.
   After treating lipid membrane-modified BPPG electrode with glutaraldehyde, it was reacted in 1 µg/mℓ solution of v-myc to which amino group had been introduced for 30 minutes at a room temperature, whereby producing an electrode immobilized with the nucleic acid probe.
b. Gene detection using electrode immobilized with nucleic acid probe

As a test sample, plasmid pVM 623 obtained by inserting v-myc (1.5 Kb) into Pst I site of pUC 119 was employed.

The test sample was thermally denaturated at 95°C and then the electrode immobilized with the nucleic acid prepared in step a was placed in the sample to effect hybridization at 55°C. Determination of the membrane potential of the electrode immobilized with the nucleic acid was started before the initiation of hybridization. The membrane potential changed as the hybridization proceeded, and plateaued about 2 hours after initiation of reaction. Thus, when using the electrode immobilized with the nucleic acid probe via the membrane, the gene can be detected while monitoring the hybridization reaction. As a result, the intended gene could be detected in the order of pg.

### Example 8: Gene detection using electrode immobilized with nucleic acid probe and blocked by synthetic oligonucleotide

a. Preparation of electrode immobilized with nucleic acid probe
   (6-aminohexyl) dATP was introduced at 3' terminal into synthetic oligonucleotide probe (20 mer) against to a carcinogenic gene v-myc using terminal deoxynuclotidyl transferase.
   On the other hand, BPPG electrode was subjected to electrolysis at 2.2 V in a solution containing 10% nitric acid and 2.5% potassium chromate to oxidize the surface of the electrode. The electrode whose surface had been oxidized was then placed in a refluxed solution of 10% gamma-APTES in aniline at 120°C for 30 minutes to effect silane treatment. After the silane-treated electrode was washed with methanol, it was reacted in 1% glutaraldehyde solution for 30 minutes, and then washed again.
   This electrode was reacted in a solution containing 1 µg/md of the probe to which amino group had been introduced at a room temperature for 30 minutes, whereby producing an electrode immobilized with the nucleic acid probe.
   This electrode was immersed in a solution of synthetic nucleotide (20 mer) to adsorb the synthetic nucleotide on the surface of electrode.
b. Gene detection using electrode immobilized with nucleic acid probe

As a test sample, pVM 623 obtained by inserting v-myc fragment into Pst I site of pUC 119 was employed. Linear pVM 623 was obtained by digesting with Hind III and then was subjected to the thermal denaturation at 98°C. The BPPG electrode immobilized with the nucleic acid probe produced in step a above was placed in the test sample, which was then incubated at 70°C to anneal. Subsequently, an intercalating agent acridine orange which binds specifically to the double stranded nucleic acid and has electrode activity was added and then the electrode reaction was conducted, during which the oxidation-reduction current generated was determined to quantify v-myc contained in the test sample.

As a result, v-myc could be detected in the order of pg. S/N ratio observed was higher than that of the electrode whose surface was not blocked.

### Example 9: Amplification and detection of intended gene

a. Amplification of intended gene
As a test sample, pVM 623 (4.6 Kb) obtained by inserting v-myc fragment into Pst I site of pUC 119 was employed. Linear pVM 623 was obtained by digesting with Hind III and then the concentration was adjusted to 1 femtomol (10⁻¹⁵ mol). The sample was subjected to PCR using the condition shown below as one cycle and repeating the cycle 30 times to amplify 1 Kb fragment of v-myc.

| | |
|---|---|
| Denaturation : | 94°C for 1 minute |
| Primer annealing : | 55°C for 1 minute |
| DNA elongation : | 72°C for 1 minute |

b. Gene detection using electrode immobilized with nucleic acid probe

The sample amplified by PCR was thermally denaturated at 98°C and BPPG electrode immobilized with nucleic acid probe was placed in the sample, which was then incubated at 70°C for 15 minutes to effect annealing. During this procedure, acridine orange, an intercalating agent which has specificity to the double stranded nucleic acid and has the electrode activity, was added to the sample. Subsequently, the electrode reaction was conducted while determining the oxidation-reduction current generated to quantify v-myc contained in the sample. As a result, the presence of v-myc could be confirmed.

### Example 10: Gene detection while inhibiting non-specific adsorption of nucleic acid to electrode immobilized with nucleic acid probe

As a test sample, pVM 623 obtained by inserting v-myc fragment into Pst I site of pUC 119 was employed. Linear pVM 623 was obtained by digesting with Hind III and then was subjected to the thermal denaturation at 98°C. The BPPG electrode immobilized with the nucleic acid probe was placed in the test sample, which was incubated at 70°C to effect annealing. After annealing, -1.5 V (vs SCE) potential was applied to the electrode to remove DNA adsorbing the electrode surface non-specifically and physically.

Then acridine orange, an intercalating agent which has specificity to the double stranded DNA and has electrode activity, was added and then the electrode reaction was conducted. The oxidation-reduction current generated was determined to quantify v-myc contained in the sample. As a result, v-myc could be detected in the order of pg at an improved S/N ratio when compared with conventional results.

### Example 11: Gene detection while inhibiting non-specific adsorption of nucleic acid to electrode immobilized with nucleic acid probe

As a test sample, pVM 623 obtained by inserting v-myc fragment into Pst I site of pUC 119 was employed. Linear pVM 623 was obtained by digesting with Hind III and then was subjected to the thermal denaturation at 98°C. BPPG electrode immobilized with the nucleic acid probe (20 mer) having 50% homology to v-myc and BPPG electrode immobilized with the nucleic acid probe against to v-myc were placed in the test sample, which was incubated at 70°C to effect annealing.

Then acridine orange, an intercalating agent which has specificity to the double stranded DNA and has electrode activity, was added and then the electrode reaction was conducted. The oxidation-reduction current generated was determined to quantify v-myc contained in the sample.

After this electrode reaction, -1.0 V (vs SCE) potential was applied to the electrode to remove the nucleic acid having less homology. After that, acridine orange was added similarly as above and then the electrode reaction was conducted while determining the oxidation-reduction current generated. The results indicated that the oxidation-reduction current detected from the intercalating agent when using the prove having 50% homology was about 50% of that observed when using the prove having 100% homology. Accordingly, it was clarified that variant genes can be detected by using this method.

### Reference Example 3: Extraction of nucleic acid using carrier immobilized with double stranded nucleic acid recognizing substance

In this example, a magnetite particle having particle size of 10 µm as a carrier and aminoacridine as a double stranded nucleic acid recognizing substance were employed.

First, the magnetite particles were washed thoroughly with PBS and placed in a refluxed solution of 10% 3-aminopropyl triethoxysilane in toluene at 120°C for 2 hours, and then washed with methanol. Subsequently, 1% glutaraldehyde solution was reacted with the particles to which then aminoacridine was immobilized.

Human leukocyte was used as a test sample. The leukocytes and the magnetic particles immobilized with aminoacridine were admixed in a plastic vessel, which was agitated vigorously by vortex mixer to effect both of cell rupture and immobilization of the nucleic acid to the carrier at once. Subsequently, a magnet was used to apply a magnetic field externally to the magnetite particles and thereby separate the particls, and the particles were washed three times with 10 mM tris buffer solution containing 200 mM NaCl (pH 7.0). After washing, the particles were placed in 70% ethanol to elute the nucleic acid.

The nucleic acid obtained was subjected to electrophoresis on 1% agarose gel, thereby obtaining the nucleic acid fragments of 20 Kb or greater, which could be cleaved with a restriction enzyme. The entire procedure could be completed within 1 hour.

As detailed above, gene detection using nucleic acid probe can be conducted conveniently within a reduced time period according to the present invention. Therefore, the present invention is extremely useful as a method for detecting a certain gene in the fields of gene diagnosis and gene engineering.

A gene detection device suitable in the present invention comprises:
a gene detection sensor having a nucleic acid probe immobilized onto the surface of a carrier sensitive to physical change;
a trnasportation means to transport the gene detection sensor;
a reaction vessel to store a sample solution containing the gene sample denatured into a single stranded form, in the reaction vessel a double stranded nucleic acid being formed on the gene sensor by hybridization of the gene sample and the nucleic acid probe immobilized on the surface of the gene sensor;
a temperature control means to control the temperature of the sample solution;
a washing means to remove unreacted gene sample by washing the gene sensor after hybridization of the nucleic acid probe with the gene sample; and,
a detection vessel to store a double stranded nucleic acid recognizing substance, the double stranded nucleic acid recognizing substance being reacted with the double stranded nucleic acid formed on the surface of the gene sensor in the detection vessel, whereby binding the double stranded nucleic acid recognizing substance with the double stranded nucleic acid in order to detect a physical change generated by the bound double stranded nucleic recognizing substance.

The gene sensor used in the said gene detection device may preferably be either of the electrode or optical fiber immobilized with the nucleic acid probe as mentioned above. This gene sensor may further be imparted with the function of a stirrer by forming it in the form of paddle or with the function of a temperature sensor.

The physical change such as electrochemical or optical signal detected by the gene sensor were determined directly or via appropriate controller and then further analyzed by means of computer.

In the reaction vessel, a sample solution containing the gene sample denaturated into a single strand was stored. As the sample solution, the coarse extract of the nucleic acid obtained by cell rupture may be used as it is or used after purification. A sample solution preparating device capable of preparing such coarse or purified extract of the nucleic acid may be connected to the reaction vessel and the sample solution prepared in situ from the test cells may be fed to the reaction vessel, whereby enabling full-automatic gene detection starting from the preparing the sample soulution from the test cells. The sample solution preparing device may be, for example of a disposable cartridge type, which may be exchanged to a new cartridge after completion of the determination. By using the cartridge freely replacable, the sample solution can always be prepared in a clean condition without washing.

The gene detection device may further be provided with a dissociation means by which double stranded nucleic acid formed on the surface of the gene sensor was dissociated into the nucleic acid probe immobilized on the gene sensor surface and the free single stranded gene sample which is removed to regenerate the gene sensor. Such dissociation means is quite desirable for the purpose of automizing the detection device since the dissociation means enables the repetitive use of the gene sensor. The dissociation means useful for the present gene detection device are the treatments with heat, bases, acids, surfactants and ultrasonic wave as mentioned above.

Furthermore, the said gene detection device may employ one or more gene sensors each immobilized with different nucleic acid probes. These gene sensors may be used at once to determine one or more items, or some of the gene sensors may be designated for a certain choice of the items to be detected.

A method of detecting the gene using the above-described gene detection device is described below with referring the figures.

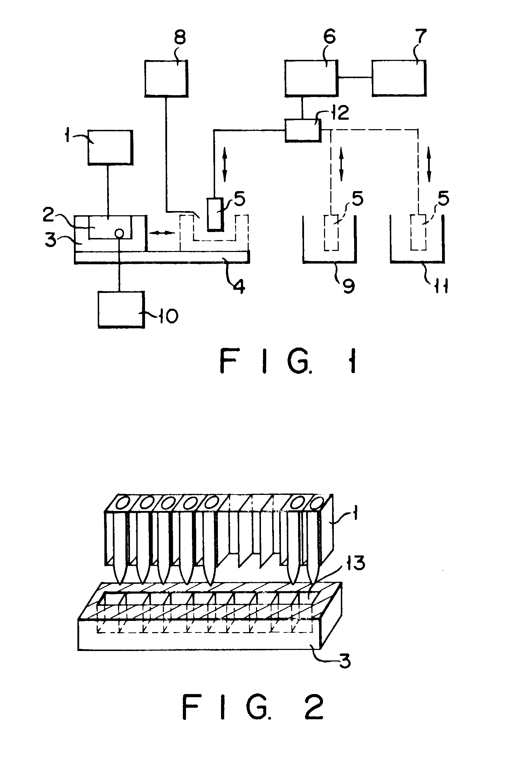

Fig. 1 shows a schematic view of an example of the said automatic gene detection device. This device comprises three vessels, namely, reaction vessel 2, detection vessel 9 and dissociation treatment vessel 11. Reaction vessel 2 is fitted in temperature controller 3 and connected to waste tank 10, and is able to move in horizontal direction along with rail 4. Reaction vessel 2 is connected to gene sample purification device 1 at a predetermined position on rail 4. Gene sensor 5 is fixed on transportating device 12, by which horizontal transportation to the position above each tank and vertical transportation into each tank are effected. As gene sensor 5, an electrode immobilized with a nucleic acid probe is employed and the electric signals detected by this electrode is input via electric signal detection controller 6 to computer 7, by which the signals are analyzed.

The method of detecting gene using this device is as follows. First, a test cells containing the nucleic acid to be detected is placed in gene sample purification device 1, in which a test solution containing the gene samples denaturated into single strands is prepared. The sample solution thus prepared is fed to reaction vessel 2, which is then transported to the predetermined position along with rail 4. After gene sensor 5 was horizontally transported to the position above reaction vessel 2, it is transported into reaction 2. After gene sensor 5 was immersed in the sample solution in reaction vessel 2, the temperature of the sample solution was appropriately controlled by temperature controller 3 and then the nucleic acid probe immobilized to the surface of gene sensor 5 and the gene sample contained in the sample solution are hybridized. After the completion of hybridization, gene sensor 5 is taken from the sample solution and washed with fluid fed from washing fluid vessel 8 to remove unreacted nucleic acid probe, and then the sensor is horizontally transported to the position above detection vessel 9. After removing gene sensor 5, reaction vessel 2 is transported again to the position connecting to gene sample purification device 1, and the sample solution contained is discharged to the waste tank. Gene sensor 5 transported to the position above detection vessel 9 is then transported into detection vessel 9. In detection vessel 9, a solution containing a double stranded nucleic acid recognizing substance is stored, and the double stranded nucleic acid recognizing substance recognizes and binds to the double stranded nucleic acid formed on the surface of gene sensor 5 immersed in the solution. Electrochemical signal generated by the double stranded nucleic acid recognizing substance bound to the surface is detected by gene sensor 5 and controlled by electric signal detection controller 6 and then input to computer 7 for analysis. After determination, gene sensor 5 is removed from detection vessel 9 and transported into dissociation treatment vessel 11. In dissociation treatment vessel 11, the double strands formed on the surface of gene sensor 5 is dissociated to regenerate gene sensor 5.

Reaction vessel 2 mentioned above may not necessarily be single, and combination of one or more small vessels 13 may also be employed as shown in Fig. 2. By using such one or more small vessels and one or more gene sensors 5, one or more samples can be determined at once. In such case, independent small tanks 13 may also be combined with the same number of gene sample purification devices 1 to prepare a plurality of samples at once, whereby enhancing the efficiency of the determination.

The determination can also be conducted without removing unreacted nucleic acid samples and double stranded nucleic acid recognizing substances. In such case, there is no need to provide washing fluid tank 8 and detection vessel 9, and the entire procedure through determination can be conducted in reaction vessel 2.

Furthermore, reaction vessel 2 may be a disposable reaction cell provided with a carrier immobilized with the nucleic acid probe on its bottom or side surface. The immobilized carrier useful may preferably be an electrode immobilized with a nucleic acid probe in view of the connection to the detection device body, although any other immobilized carrier listed above can be used. It is also preferable to set the immobilized carrier such a manner that it can be separated from the reaction cell for the purpose of repetitive use.

The gene detection using this reaction cell is conducted as follows. First, a sample solution containing the nucleic acid to be detected is placed in the reaction cell, which is heated to denaturate the nucleic acid to the single strand. Annealing is then conducted at a temperature selected suitably depending on the probe employed to form the double strand. The double stranded nucleic acid recognizing substance was added and the signal generated directly or indirectly is determined via the carrier provided in the reaction cell. In such case, it is not required to use the gene sensor mentioned above.

The reaction cell is separated from the detection device and discarded after every determination. Therefore, the gene detection highly reliable without cross-contamination or carry-over of the samples. In addition, the determination can be conducted more simply within a short period since there is no need to wash the cell.

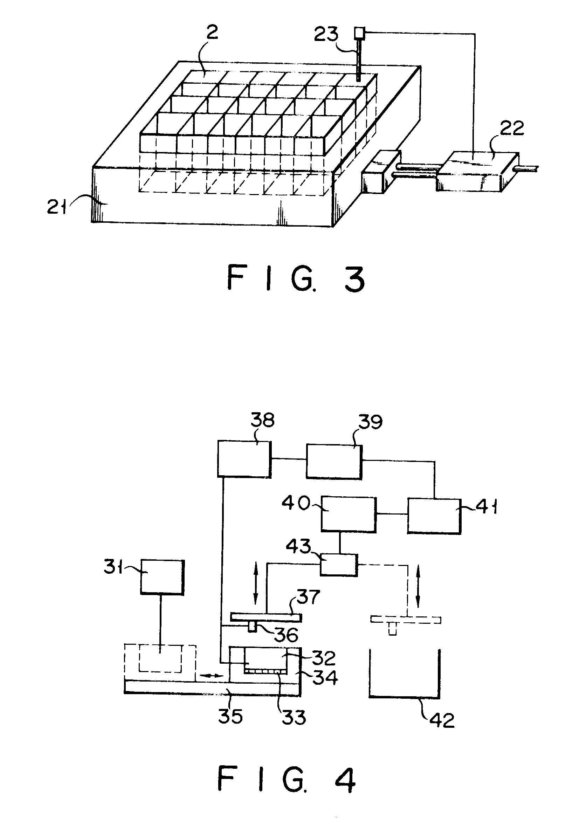

As shown in Fig. 3, temperature controller 3 which controls the temperature of reaction vessel 2 has thermostat bath 21, controller 22 to control the temperature of thermostat bath 21, and temperature sensor 23 to determine the temperature of the sample solution. In Fig. 3, reaction vessel 2 provided in thermostat bath 21 consists of one or more small vessel 13 mentioned above. One of small vessels 13 contains a buffer solution having the composition same as that of a solution used in the sample solution, and temperature sensor 23 is inserted the buffer solution. The temperature of the buffer solution is determined as the temperature of the sample solution. Temperature sensor 23 is connected to controller 22, and it determines the temperature of the buffer solutlon in reaction vessel 2 and sends the data to controller 22. After receiving the temperature data from temperature sensor 23, controller 22 calculate and processes the data and then control the temperature of thermostat bath 21 to maintain the temperature of the sample solution at a certain constant level. This temperature control is preferably conducted within the range of ±0.5°C.

An example of the automatic gene detection device utilizing electrochemiluminescence is described below.

Fig. 4 shows a schematic view of an automatic gene detection device utilizing electrochemiluminescence. This device is provided with reaction cell 32 having the functions of both of reaction and detection vessels in the detection device shown in Fig. 1 and washing vessel 42. As mentioned above, it is not required to provide a reaction vessel or detection vessel separately since the determination can be conducted without removing unreacted nucleic acid probe or unreacted intercalating agent when using electrochemiluminescence. On the bottom surface of reaction cell 32, electrode 33 immobilized with a nucleic acid probe is provided. Similarly as in the reaction vessel of the detection device shown in Fig. 1, reaction cell 32 is fitted to temperature controller 34. The reaction cell is transported horizontally along with rail 35 and connected to gene sample purification device 31 at a predetermined position of rail 35. Reference electrode 36 is fixed to transporting device 12 together with the tip of optical fiber 37. By transporting device 12, reference electrode 36 and optical fiber 37 are horizontally transported and vertically transported into each vessel. Reference electrode 36 is connected to function generator/potentiostat 38 together with electrode 33 immobilized with nucleic acid probe. The potential to be applied to these electrodes is appropriately calculated by computer 39. The electrochemiluminescence generated on the surface of electrode 33 immobilized with nucleic acid probe is sent via optical fiber 37 to photomultiplier 40 for amplification and then counted by photocounter 41. The results of the determination are input to computer 39 for analysis.

The gene detection using this device is conducted as follows. First, similarly as in the case of the device shown in Figure 1, the test cells containing the nucleic acid to be detected are placed in gene sample purification device 31 and processed into a sample solution containing the gene sample denaturated to the single strand. The solution is then transferred to reaction cell 32. Then, the temperature of the sample solution is controlled appropriately by temperature controller 34 and the nucleic acid immobilized on the surface of electrode 33 is hybridized with the gene sample in the sample solution. During this procedure, an intercalating agent capable of generating electrochemiluminescence is added to the sample solution. Alternatively, the intercalating agent may be added to the test solution prior to the hybridization. Subsequently, reaction cell 32 is transported along with rail 35 to a predetermined position and reference electrode 36 and optical fiber 37 are transported into reaction cell 32 to immerse in the sample solution. Then potential is applied between reference electrode 36 and electrode 33 immobilized with the nucleic acid provided in reaction cell 32 to generate electrochemiluminescence. The light caused by the electrochemiluminescence is brought via optical fiber 37 to photomultiplier 40 for amplification followed by counting by photocounter 41. The results of the determination are input to computer 39 for analysis. After determination, reference electrode 36 and optical fiber 37 are removed from reaction cell 32 and transferred to washing vessel 42 for washing.

### Example 12: Gene detection using gene detection device provided with electrode immobilized with nucleic acid probe

The automatic gene detection device shown in Fig. 1 was used to detect the gene. As a test sample, pVM 623 obtained by inserting v-myc fragment into Pst 1 site of pUC 119 was employed. Linear pVM 623 was obtained by digesting with Hind III and then was subjected to the thermal denaturation at 98°C. The electrode immobilized with the nucleic acid probe was placed in the test sample, which was incubated for 15 minutes at 70°C to effect annealing. During this procedure, acridine orange, an intercalating agent which has specificity to the double stranded DNA and is electrochemically active, was added.

After the annealing, the electrode reaction was effected while determining the oxidation-reduction current produced to qunntify v-myc contained in the test sample. As a result, v-myc could be detected in pg order. The entire procedure could be conducted within 1 hour.

### Reference Example 4: Gene detection using gene detection device provided with electrode immobilized with nucleic acid probe utilizing electrochemiluminescence

The gene was detected by using the automatic gene detection device shown in Fig. 4. As a test sample, DNA derived from human peripheral blood admixed with pVM 623 obtained by inserting v-myc fragment into Pst I site of pUC 119 and digested with Hind III to make it linear was employed. A reaction cell provided on its bottom surface with a BPPG electrode immobilized with nucleic acid probe was prepared, and then the sample was added to this reaction cell and thermally denaturated for 5 minutes at 98°C. The sample was incubated for 15 minutes at 70°C to effect annealing. During annealing, lucigenin which has specificity to the double stranded DNA and capable of generating electrochemiluminescence was added.

After the annealing, the electrochemical reaction was effected while counting the luminescent light by a photo-counter. As a result, v-myc could be detected in pg order. The entire procedure could be conducted within 1 hour.

### Example 13: Gene detection using disposable reaction cell provided with electrode immobilized with nucleic acid probe

a. Preparation of reaction cell provided with electrode immobilized with nucleic acid probe
   (6-aminohexyl) dATP was introduced to carcinogenic gene v-myc (1.5 Kb) previously at 3' terminal using terminal deoxynuclotidyl transferase.
   As an electrode to be immobilized with the nucleic acid, a Pt electrode was employed. First, the Pt electrode was heated at 180°C for 12 hours to oxidize the surface of the electrode. The electrode was placed in a refluxed solution of 10% gamma-aminopropyl triethoxy silane in aniline at 120°C for 30 minutes to effect silane treatment and then washed. The electrode was reacted further in 1% glutaraldehyde solution for 30 minutes and then washed. Subsequently, the electrode was reacted in a solution containing 1 µg/mℓ v-myc having amino group introduced at a room temperature for 30 minutes, whereby producing an electrode immobilized with the nucleic acid probe. Then a reaction cell (5 × 5 × 10 mm) provided on its bottom surface with the electrode immobilized with the nucleic acid probe was prepared.
b. Gene detection using reaction cell provided with electrode immobilized with nucleic acid probe

As a test sample, the fragment obtained by digesting pVM 623 (4.6 Kb) obtained by inserting v-myc fragment into Pst I site of pUC 119 with Hind III was employed. A solution containing the segment was placed in the reaction cell produced in step a, and subjected to thermal denaturation at 95°C for 5 minutes followed by annealing at 72°C for 30 minutes. After reaction, tris (1,10-phenanthroline) osmium was added to the reaction cell and the electrochemiluminescence caused by applying potential to the electrode was determined. As a result, v-myc could be determined in the order of pg.

### Example 14: Gene detection using disposable reaction cell provided with electrode immobilized with nucleic acid probe

a. Preparation of reaction cell provided with electrode immobilized with nucleic acid probe
   (6-aminohexyl) dATP was introduced to carcinogenic gene v-myc (1.5 Kb) previously at 3' terminal using terminal deoxynuclotidyl transferase.
   As an electrode to be immobilized with the nucleic acid, BPPG electrode was used. First, this BPPG electrode was electrolyzed in a solution containing 10% nitric acid and 2.5% potassium chromate at 2.2 V to oxidize the surface of the electrode. The electrode was placed in a refluxed solution of 10% gamma-aminopropyl triethoxy silane in aniline at 120°C for 30 minutes to effect silane treatment and then washed with methanol. The electrode was reacted further in 1% glutaraldehyde solution for 30 and then washed. Subsequently, the electrode was reacted in a solution containing 1 µg/mℓ v-myc having amino group introduced at a room temperature for 30 minutes, whereby producing an electrode immobilized with the nucleic acid probe. Then a reaction cell (5 × 5 × 10 mm) provided on its bottom surface with the electrode immobilized with the nucleic acid probe was prepared.
b. Gene detection using reaction cell provided with electrode immobilized with nucleic acid probe.

As a test sample, the fragment obtained by digesting pVM 623 (4.6 Kb) obtained by inserting v-myc fragment into Pst I site of pUC 119 with Hind III was employed. A solution containing the fragment was placed in the reaction cell produced in step a, and subjected to thermal denaturation at 95°C for 5 minutes followed by annealing at 72°C for 30 minutes. After reaction, tris (1,10-phenanthroline) cobalt was added to the reaction cell and the oxidation-reduction potential was determined by cyclic voltammetry. As a result, v-myc could be determined in the order of pg.

As detailed described above, by using the automatic gene detection device according to the present invention, the gene can be detected automatically by the methods mentioned above. Therefore, the gene can be detected more simply within a short period.

In the detection method described above, for the purpose of providing a gene detection method capable of detecting the presence of an intended gene more safely and conveniently within a shorter period, the carriers such as an electrode or optical fiber which can detect a physical change such as electrochemical or optical signal generated from an intercalating agent is immobilized with the nucleic acid probe to use as a gene sensor. This purpose can also be achieved by using particles immobilized with the nucleic acid probe on the surface of the particles instead of the carrier immobilized with the nucleic acid probe. Thus, on the surface of the particles, the nucleic acid probe is hybridized with the gene sample to form the double strand, to which electrochemically or optically active double stranded nucleic acid recognizing substance is bound, and then the double stranded nucleic acid recognizing substance is detected electrochemically or optically by a detector.

The particles to be immobilized with the nucleic acid probe may be, but are not limited to, latex beads, polystyrene beads, glass beads, magnetic particles and the like. The diameter of the particle employed is preferably within the range from 100 A to 1 mm.

Other conditions are similar to those in cases of using the carrier such as the electrode or optical fiber immobilized with the nucleic acid probe as mentioned above.

Similarly, a filter immobilized with the nucleic acid probe on the filter surface may also be employed to detect the gene. In such case, the useful filters may be any filters as long as they do not change their nature at least at 100°C, such as, for example, those filters generally used in DNA southern blotting such as nitrocellulose filters and nylon filters. For immobilization of the nucleic acid probe onto those filters, the methods mentioned above for immobilization of the nucleic acid to a carrier can be applied as they are.

Gene detection using the filter immobilized with the nucleic acid probe can be conducted as follows.

First, a nucleic acid is extracted from a test sample such as peripheral venous blood or various organ cells according to a standard method and purified if necessary. Then a hybridization reaction solution containing the nucleic acid sample obtained is prepared and the reaction solution is applied to the multiple-layer filter device consisting of one or more filters including the filter immobilized with the nucleic acid probe, and the sample is permeated into the filter device. A double stranded nucleic acid recognizing substance, especially such a double stranded nucleic acid recognizing substance that indicates optical activity itself or via other substance, has previously been contained in the hybridization reaction solution. After the reaction solution is sufficiently permeated, the nucleic acid is thermally denaturated at 95°C into a single strand, which is then hybridized with the nucleic acid probe immobilized on the filter surface by further heating at 37 to 72°C. After reaction, the filter immobilized with the nucleic acid probe is removed from the filter device and then washed. When the intended gene is present in the nucleic acid sample, a double strand is formed on the filter immobilized with the nucleic acid probe and this double stranded nucleic acid is bound to the double stranded nucleic acid recognizing substance. This double stranded nucleic acid recognizing substance causes the change in signal, which is determined to quantify the intended gene. Thus, when the double stranded nucleic acid recognizing substance has optical activity, then the change in optical signal such as luminescence, fluorescence, reflection, fluorescence polarization, quenching, circular dichroism and the like.

Most of the genetic diseases are developed when a particular base sequence is defected or combination of a plurality of particular base sequences are presented in a gene. Thus, most of the genetic diseases can not be identified directly by using the nucleic acid probe. Accordingly, most of the diseases are now identified by using restriction fragments length polymorphism (RFLP) analysis. This RFLP method involves an analysis of the pattern of DNA fragments and thus requires the procedure of separating the DNA fragments. Presently, the separation of DNA fragments employs only electrophoresis which involves the problem of complicated procedures and a long time period for the determination.

Such analysis of the pattern of DNA fragments can be conducted conveniently within a short period by using the gene detection method of the present invention in the procedure described below.

First, DNA is extracted from a biological material and then digested with an appropriate restriction enzyme. The restriction enzyme useful is not particularly limited and any enzyme generally used in RFLP may be employed. Examples of the useful restriction enzyme are AccI, AvaI, BamHI, EcoRI, HincI, HndIII and Pst I.

The DNA fragments obtained are then separated based on molecular weights by column chromatography, high performance liquid chromatography (HPLC), for example, FPLC® (manufactured by PHARMACIA Co. Ltd.), capillary electrophoresis, gel electrophoresis and the like. DNA fragment may also be separated after denaturated by, for example, heating at 90 to 98°C.

Then DNA fragments separated based on molecular weights are hybridized with the nucleic acid probe immobilized onto the carrier. This hybridization may be conducted in a flow system of a constant flow rate or in a series of fractions having the same volume.

When the hybridization is conducted in the flow system, the conditions such as temperature and pH of moving phase are suitably selected depending on the hybridization reaction. The composition of the moving phase is not particularly limited although a salt concentration of about 0 to 1 M, pH of the neutral region, and a temperature within the range from 37 to 72°C are preferred. While it is desirable to add the double stranded nucleic acid recognizing substance previously to the sample solution, it is also acceptable that a carrier having the double strands formed on the surface is placed in a solution containing the double stranded nucleic acid recognizing substance. As a double stranded nucleic acid recognizing substance, any of those listed above may be employed. In the flow system, the time period (retention time) between the introduction of the sample solution and the generation of the direct or indirect signal from the double stranded nucleic acid recognizing substance is determined. The pattern of DNA fragments based on, for example, RFLP can be obtained.

When the hybridization is conducted after obtaining the fractions, each fraction is subjected to the hybridization with the nucleic acid probe immobilized to the carrier and then the double stranded nucleic acid recognizing substance is added to the fraction to determine the direct or indirect signal from the double stranded nucleic acid recognizing substance. From the fraction number of the fraction in which the signal is obtained, the pattern of DNA fragments based on, for example, RFLP can be obtained.

Although the present invention is a method to detect the presence of a gene having a particular base sequence as mentioned above, the present method can further enable the separation of the gene having the particular base sequence. Specifically, since in the detection method mentioned above the intended gene is immobilized to a carrier by means of hybridization with the nucleic acid probe, the intended gene can be separated from the test sample only by removing the carrier from the test sample. Accordingly, the intended gene can exclusively separated by dissociating the intended gene from the carrier by an appropriate means after removing the substrate.

More specifically, this gene separation can be performed by first hybridizing the nucleic acid probe immobilized on the carrier gene surface with the intended gene to form a double stranded nucleic acid, to which then the double stranded nucleic acid recognizing substance added before or after hybridization is bound, and then by detecting the signal from the double stranded nucleic acid recognizing substance bound to the double stranded nucleic acid to confirm the presence of the intended gene. Then the carrier on which the presence of the intended gene has been confirmed is removed from the test sample to separate the intended gene from the test sample, and then by means of thermal or alkaline denaturation the intended gene is dissociated from the carrier. In this gene separation method, the procedure to the step of detecting the intended gene contained in the test sample is similar to that in the gene detection method mentioned above.

To dissociate the intended gene from the carrier, the carrier may be heated at a temperature of 95°C or higher in a buffer solution or may be exposed to an alkaline condition using for example sodium hydroxide. By this procedure, the intended gene is separated in a form of a single strand.

The gene thus obtained is converted to a double strand by synthesizing a complementary chain using an enzyme. The yield may be increased by amplification using an enzyme. Especially by PCR method, the yield can be increased while forming a double strand at a same time. By forming a double strand which is then incorporated via a linker into a vector, the intended gene can be cloned efficiently and easily.

### Example 15: Indirect gene detection by RFLP analysis using electrode immobilized with nucleic acid probe

Using an electrode immobilized with the nucleic acid probe, the individual identification by DNA fingerprint method was conducted as follows.

First, leukocytes were separated from human peripheral venous blood by density gradient centrifugation and DNA was extracted according to a standard method. Then DNA obtained was digested with a restriction enzyme Hae III.

On the other hand, Myo probe was immobilized to BPPG electrode to prepare an electrode immobilized with the nucleic acid probe, which was then fitted to the outlet of the column of high pressure liquid chromatograph (HPLC). By loading the sample to HPLC, DNA including the sequence having homology to the probe immobilized to the electrode surface was supported temporarily on the electrode surface to form a double strand, to which then the double stranded nucleic acid recognizing substance such as an intercalating agent was bound, whereby enabling the determination of electrochemical signal from the double stranded nucleic acid recognizing substance. The signals obtained exhibit a specific pattern. By analyzing this pattern, the patterning of the sample can be conducted.

By thermally denaturating the fragment of DNA obtained as above at 95°C to obtain a single strand, which was then subjected to HPLC together with a intercalating agent, acridine orange, to conduct the patterning of DNA. During chromatography, the column was maintained at 75°C.

The results obtained indicated the discrete patterns by individuals subjected to the study. Accordingly, it was clearly indicated that individual identification is possible by the present gene detection method.

### Reference Example 5: Gene separation using electrode immobilized with nucleic acid probe

A solution of chromosomal DNA (10 µg/mℓ) extracted from E. coil strain JM 109 admixed with of 1 µg/mℓ digested pVM 623 (obtained by inserting v-myc to pUC 119) with Hind III was used as an experimental model system. The sequence 5'-TGCAGTTCCGGTGGCTGATC-3' in v-myc was used as a probe for detection and separation.
a. Preparation of BPPG electrode immobilized with nucleic acid probe
   BPPG electrode was electrolyzed in a solution of 2.5% potassium chromate and 10% nitric acid at 2.2 V for 10 seconds to oxidize the surface. The electrode was placed in a refluxed solution of 10% gamma-aminoprpyltriethoxy silane in toluene at 120°C for 30 minutes to effect silane treatment. By this treatment, amino group was introduced to the electrode surface. Then the electrode was allowed to stand in 1/15 M phosphate buffer (pH 7.0) containing 1% glutaradldehyde for 1 hour at a room temperature to introduce aldehyde group. The synthetic primer mentioned above was prepared into a solution of 10 µg/mℓ in 10 mM phosphate buffer, in which the electrode treated with aldehyde was then placed and allowed to stand for 1 hour at a room temperature. In this step, the primer was immobilized on the electrode surface.
b. Detection of intended gene
   The BPPG electrode immobilized with the nucleic acid probe prepared in step a was placed in a solution of chromosomal DNA (10 µg/mℓ) extracted from E. coil strain JM 109 admixed with of 1 µg/mℓ of pVM 623 which had been converted to a linear form by using Hind III and was hybridized at 55°C. An intercalating agent, acridine orange, was added to obtain the concentration of 1 µM and then the electrode response was determined.
   As a result, the peaks specific to acridine orange were obtained, indicating the formation of the double strands on the electrode surface.
c. Separation of intended gene

The electrode was removed from the sample solution and heated at 95°C in the buffer to dissociate the intended gene in a double strand form. Then 5'-TGCAGTTCCGGTGGCTGATC-3' and 5'-CGACTCGGAAGAAGAACAAG-3' which were the fragments in v-myc were used as the primers to conduct PCR. The length between the base sequences complementary to the two primers is about 900 bp. The gene amplified by PCR was subjected to electrophoresis, in which the band corresponding to 900 bp was observed, whereby confirming the separation of pVM 623 which was intended to be separated.

As a control, the same procedure was taken using pUC 118 as an intended gene and the base sequence in pUC 118 as a PCR primer, and no band was observed at the corresponding position.

### Example 24: Gene detection using electrode immobilized with nucleic acid probe and blocked by stearylamine

a. Preparation of electrode immobilized with nucleic acid probe
   (6-aminohexyl) dATP was introduced at 3' terminal into synthetic oligonucleotide probe (20 mer) against to a carcinogenic gene v-myc using terminal deoxynuclotidyl transferase.
   On the other hand, BPPG electrode was subjected to electrolysis at 2.2 V in a solution containing 10% nitric acid and 2.5% potassium chromate to oxidize the surface of the electrode. The electrode whose surface had been oxidized was then placed in a refluxed solution of 10% gamma-APTES in aniline at 120°C for 30 minutes to effect silane treatment. After the silane-treated electrode was washed with methanol, it was reacted in 1% glutaraldehyde solution for 30 minutes, and then washed again.
   This electrode was reacted in a solution containing 1 µg/mℓ of the probe to which amino group had been introduced at a room temperature for 30 minutes, whereby producing an electrode immobilized with the nucleic acid probe.
   This electrode was immersed in a stearylamine solution to adsorb stearyamine on the surface of electrode, thereby a non-specific adsorption of a sample was suppressed.
b. Gene detection using electrode immobilized with nucleic acid probe

As a test sample, pVM 623 obtained by inserting v-myc fragment into Pst I site of pUC 119 was employed. Linear pVM 623 was obtained by digesting with Hind III and then was subjected to the thermal denaturation at 98°C. The BPPG electrode immobilized with the nucleic acid probe produced in step a above was placed in the test sample, which was then incubated at 70°C to anneal. Subsequently, an intercalating agent acridine orange which binds specifically to the double stranded nucleic acid and has electrode activity was added and then the electrode reaction was conducted, during which the oxidation-reduction current generated was determined to quantify v-myc contained in the test sample.

As a result, v-myc could be detected in the order of pg. S/N ratio observed was higher than that of the electrode whose surface was not blocked.

## Claims

1. A gene detection method wherein a single stranded nucleic acid probe having a base sequence complementary to the gene to be detected is reacted with a gene sample denatured into a single stranded form and then said nucleic acid probe hybridized with the gene is detected to confirm the presence of the gene, characterized in that:
said nucleic acid probe is immobilized onto an electrode;
a double stranded acid recognizing substance capable of binding specifically to a double stranded nucleic acid and being active electrochemically is added to the reaction system of said nucleic acid probe and the gene sample, said double stranded nucleic acid recognizing substance being an intercalating agent; and,
the double stranded nucleic acid recognizing substance bound to the double stranded nucleic acid formed by conjugation of said nucleic probe and the gene to be detected is detected by means of electrochemical determination using said electrode, whereby detecting the presence of said nucleic acid probe hybridized with the gene to be detected.

2. A method according to claim 1, wherein potential is applied to the electrode before and/or upon hybridization of the nucleic acid probe immobilized on the surface of the electrode and the gene sample denatured into a single stranded form.

3. A method according to claim 1 or 2, wherein potential is applied to the electrode after hybridization of the nucleic acid probe immobilized on the surface of the electrode and the gene sample denatured into a single stranded form.

4. A method according to any preceding claim, wherein said intercalating agent is a compound selected from the group consisting of ethidium, ethidium bromide, acridine, aminoacridine, acridine orange, proflavin, ellipticine, actinomycin D, daunomycin and mitomycin C.

5. A method according to any of claims 1 to 3, wherein said intercalating agent is a metal complex having as a centre metal a metal capable of undergoing electrically reversible oxidation-reduction reaction and the oxidation-reduction potential of said metal is less than or is not covered by the oxidation-reduction potential of the nucleic acid.

6. A method according to claim 5, wherein said metal complex is a compound selected from the group consisting of tris (phenanthroline) zinc salt, tris (phenanthroline) ruthenium salt, tris (phenanthroline) cobalt salt, di (phenthroline) zinc salt, di (phenanthroline) ruthenium salt, di (phenanthroline) cobalt salt, bipyridine cobalt salt, terpyridine platinum salt, phenanthroline platinum salt, tris (bipyridyl) zinc salt, tris (bipyridyl) ruthenium salt, tris (bipyridyl) cobalt salt, di (bipyridyl) zinc salt, di (bipyridyl) ruthenium salt, di (bipyridyl) cobalt salt.

7. A method according to any of claims 1 to 3, wherein said double stranded nucleic acid recognizing substance is an intercalating agent to which one or more substances generating electric signals which can be detected by said electrode directly or indirectly are bound.

8. A method according to any one of claims 1 to 7, wherein said nucleic acid probe is immobilized onto the electrode through an amino group introduced in said nucleic acid probe.

9. A method according to any one of claims 1 to 7, wherein said nucleic acid probe is immobilized onto the electrode through a film.

10. A method according to any of claims 1 to 9, wherein said electrode is further covered with substances selected from the group consisting of nucleic acids, surfactants, fatty acids and fats.

11. A gene detection method wherein a single stranded nucleic acid probe having a base sequence complementary to the gene to be detected is reacted with a gene sample denatured into a single stranded form and then said nucleic acid probe hybridized with the gene is detected to confirm the presence of the gene, characterized in that:
said nucleic acid probe is immobilized onto an electrode;
an intercalating agent capable of binding specifically to a double stranded nucleic acid and exhibiting electrochemiluminescence is added to the reaction system of said nucleic acid probe and the gene sample; and,
the intercalating agent bound to the double stranded nucleic acid formed by conjugation of said nucleic acid probe and the gene to be detected is detected by means of determination of optical signals generated by the electrochemiluminescence using a photo detector separately existed from the electrode in the reaction system, whereby detecting the presence of said nucleic acid probe hybridized with the gene to be detected.

## Patentansprüche

1. Gennachweisverfahren, bei welchem eine einzelsträngige Nucleinsäuresonde mit einer zu dem nachzuweisenden Gen komplementären Basensequenz mit einer zu einer einzelsträngigen Form denaturierten Genprobe zur Reaktion gebracht und danach die mit dem Gen hybridisierte Nucleinsäuresonde zur Bestätigung der Anwesenheit des Gens nachgewieseb wird, dadurch gekennzeichnet, daß die Nucleinsäuresonde auf einer Elektrode immobilisiert wird;
eine eine doppelsträngige Nucleinsäure erkennende Substanz mit der Fähigkeit zur spezifischen Bindung an eine doppelsträngige Nucleinsäure und elektrochemischer Aktivität zu dem Reaktionssystem aus der Nucleinsäuresonde und der Genprobe zugegeben wird, wobei es sich bei der eine doppelsträngige Nucleinsäure erkennenden Substanz um ein interkalierendes Mittel handelt, und die eine doppelsträngige Nucleinsäure erkennende Substanz, welche an die durch Konjugation der Nucleinsäuresonde und des nachzuweisenden Gens gebildete doppelsträngige Nucleinsäure gebunden ist, auf elektrochemischem Wege unter Verwendung der obigen Elektrode bestimmt wird, wobei das Vorhandensein der mit dem nachzuweisenden Gen hybridisierten Nucleinsäuresonde nachgewiesen wird.

2. Verfahren nach Anspruch 1, wobei an die Elektrode vor und/oder nach der Hybridisierung der auf der Oberfläche der Elektrode immobilisierten Nucleinsäuresonde mit der zu einer einzelsträngigen Form denaturierten Genprobe ein Potential angelegt wird.

3. Verfahren nach Anspruch 1 oder 2, wobei an die Elektrode nach der Hybridisierung der auf der Oberfläche der Elektrode immobilisierten Nucleinsäuresonde mit der zu einer einzelsträngigen Form denaturierten Genprobe ein Potential angelegt wird.

4. Verfahren nach einem der vorhergehenden Ansprüche, wobei es sich bei dem interkalierenden Mittel um eine Verbindung, ausgewählt aus der Gruppe Ethidium, Ethidiumbromid, Acridin, Aminoacridin, Acridinorange, Proflavin, Ellipticin, Actinomycin D, Daunomycin und Mitomycin C, handelt.

5. Verfahren nach einem der Ansprüche 1 bis 3, wobei es sich bei dem interkalierenden Mittel um einen Metallkomplex mit einem Metall mit der Fähigkeit, eine elektrisch reversible Oxidations-Reduktions-Reaktion einzugehen, als zentralem Metall handelt und das Oxidations-Reduktions-Potential dieses Metalls unter dem Oxidations-Reduktions-Potential der Nucleinsäure liegt oder durch letzteres nicht abgedeckt wird.

6. Verfahren nach Anspruch 5, wobei es sich bei dem Metallkomplex um eine Verbindung, ausgewählt aus der Gruppe Tris(phenanthrolin)zinksalz, Tris(phenanthrolin)rutheniumsalz, Tris(phenanthrolin)kobaltsalz, Di(phenthrolin)zinksalz, Di(phenanthrolin)rutheniumsalz, Di(phenanthrolin)kobaltsalz, Bipyridinkobaltsalz, Terpyridinplatinsalz, Phenanthrolinplatinsalz, Tris(bipyridyl)zinksalz, Tris(bipyridyl)rutheniumsalz, Tris(bipyridyl)kobaltsalz, Di(bipyridyl)zinksalz, Di(bipyridyl)rutheniumsalz und Di(bipyridyl)kobaltsalz handelt.

7. Verfahren nach einem der Ansprüche 1 bis 3, wobei es sich bei der eine doppelsträngige Nucleinsäure erkennenden Substanz um ein interkalierendes Mittel, an das eine oder mehrere, durch die Elektrode direkt oder indirekt nachweisbare elektrische Signale erzeugende Substanz(en) gebunden ist (sind), handelt.

8. Verfahren nach einem der Ansprüche 1 bis 7, wobei die Nucleinsäuresonde auf der Elektrode über eine in die Nucleinsäuresonde eingeführte Aminogruppe immobilisiert ist.

9. Verfahren nach einem der Ansprüche 1 bis 7, wobei die Nucleinsäuresonde auf der Elektrode über einen Film immobilisiert ist.

10. Verfahren nach einem der Ansprüche 1 bis 9, wobei die Elektrode weiterhin mit Substanzen, ausgewählt aus der Gruppe Nucleinsäuren, Netzmittel, Fettsäuren und Fette bedeckt ist.

11. Gennachweisverfahren, bei welchem eine einzelsträngige Nucleinsäuresonde mit einer zu dem nachzuweisenden Gen komplementären Basensequenz mit einer zu einer einzelsträngigen Form denaturierten Genprobe zur Reaktion gebracht und danach die mit dem Gen hybridisierte Nucleinsäuresonde zur Bestätigung der Anwesenheit des Gens nachgewiesen wird, dadurch gekennzeichnet, daß die Nucleinsäuresonde auf einer Elektrode immobilisiert wird;
zu dem Reaktionssystem aus der Nucleinsäuresonde und der Genprobe ein interkalierendes Mittel mit der Fähigkeit zur spezifischen Bindung an eine doppelsträngige Nucleinsäure und Elektrochemielumineszenzeigenschaften zugegeben wird, und
das interkalierende Mittel, das an die durch Konjugation der Nucleinsäuresonde und des nachzuweisenden Gens gebildete doppelsträngige Nucleinsäure gebunden ist, durch Bestimmung von durch die Elektrochemielumineszenz erzeugten optischen Signalen unter Verwendung eines getrennt von der Elektrode im Reaktionssystem vorhandenen Photodetektors bestimmt wird, wobei das Vorhandensein der mit dem nachzuweisenden Gen hybridisierten Nucleinsäuresonde bestimmt wird.

## Revendications

1. Procédé de détection de gènes dans lequel on fait réagir une sonde acide nucléique simple brin ayant une séquence de bases complémentaire au gène à détecter avec un échantillon de gènes dénaturés en une forme simple brin, puis on détecte ladite sonde acide nucléique hybridée avec le gène pour confirmer la présence du gène, caractérisé en ce que:
ladite sonde acide nucléique est immobilisée sur une électrode;
on ajoute au système réactionnel de ladite sonde acide nucléique et de l'échantillon de gènes une substance reconnaissant les acides nucléiques double brin, capable de se lier de façon spécifique à un acide nucléique double brin et ayant une activité électrochimique, ladite substance reconnaissant les acides nucléiques double brin étant un agent intercalant; et
on détecte la substance reconnaissant les acides nucléiques double brin liée à l'acide nucléique double brin formé par la conjugaison de ladite sonde nucléique et du gène à détecter au moyen d'une mesure électrochimique utilisant ladite électrode, grâce à quoi on détecte la présence de ladite sonde acide nucléique hybridée avec le gène à détecter.

2. Procédé selon la revendication 1, dans lequel on applique un potentiel à l'électrode avant et/ou au moment de l'hybridation de la sonde acide nucléique immobilisée sur la surface de l'électrode et de l'échantillon de gènes dénaturés en une forme simple brin.

3. Procédé selon la revendication 1 ou 2, dans lequel on applique un potentiel à l'électrode après l'hybridation de la sonde acide nucléique immobilisée sur la surface de l'électrode et de l'échantillon de gènes dénaturés en une forme simple brin.

4. Procédé selon l'une quelconque des revendications précédentes, dans lequel ledit agent intercalant est un composé choisi dans le groupe constitué par l'éthidium, le bromure d'éthidium, l'acridine, l'aminoacridine, l'orangé d'acridine, la proflavine, l'ellipticine, l'actinomycine D, la daunomycine et la mitomycine C.

5. Procédé selon l'une quelconque des revendications 1 à 3, dans lequel ledit agent intercalant est un complexe métallique ayant comme métal central un métal capable de subir une réaction d'oxydoréduction électriquement réversible, et le potentiel d'oxydoréduction dudit métal est inférieur ou n'est pas couvert par le potentiel d'oxydoréduction de l'acide nucléique.

6. Procédé selon la revendication 5, dans lequel ledit complexe métallique est un composé choisi dans le groupe constitué par un sel de tris(phénanthioline)zinc, un sel de tris(phénanthroline)ruthénium, un sel de tris(phénanthroline)cobalt, un sel de di(phénanthroline)zinc, un sel de di(phénanthroline)ruthénium, un sel de di(phénanthroline)cobalt, un sel de bipyridine-cobalt, un sel de terpyridine-platine, un sel de phénanthroline-platine, un sel de tris(bipyridyle)zinc, un sel de tris(bipyridyle)ruthénium, un sel de tris(bipyridyle)cobalt, un sel de di(bipyridyle)zinc, un sel de di(bipyridyle)ruthénium, un sel de di(bipyridyle)cobalt.

7. Procédé selon l'une quelconque des revendications 1 à 3, dans lequel ladite substance reconnaissant les acides nucléiques double brin est un agent intercalant auquel sont liées une ou plusieurs substances productrices de signaux électriques pouvant être détectés directement ou indirectement par ladite électrode.

8. Procédé selon l'une quelconque des revendications 1 à 7, dans lequel ladite sonde acide nucléique est immobilisée sur l'électrode par l'intermédiaire d'un groupe amino introduit dans ladite sonde acide nucléique.

9. Procédé selon l'une quelconque des revendications 1 à 7, dans lequel ladite sonde acide nucléique est immobilisée sur l'électrode par l'intermédiaire d'un film.

10. Procédé selon l'une quelconque des revendications 1 à 9, dans laquelle ladite électrode est en outre recouverte de substances choisies dans le groupe constitué par des acides nucléiques, des agents tensioactifs, des acides gras et des graisses.

11. Procédé de détection de gènes dans lequel on fait réagir une sonde acide nucléique simple brin sonde ayant une séquence de bases complémentaire au gène à détecter avec un échantillon de gènes dénaturés en une forme simple brin, puis on détecte ladite sonde acide nucléique hybridée avec le gène pour confirmer la présence du gène, caractérisé en ce que:
ladite sonde acide nucléique est immobilisée sur une électrode;
on ajoute au système réactionnel de ladite sonde acide nucléique et de l'échantillon de gènes un agent intercalant capable de se lier de façon spécifique à un acide nucléique double brin et présentant une électrochimiluminescence; et
on détecte l'agent intercalant lié à l'acide nucléique double brin formé par la conjugaison de ladite sonde nucléique et du gène à détecter en déterminant les signaux optiques produits par l'électrochimiluminescence à l'aide d'un photodétecteur séparé de l'électrode dans le système réactionnel, grâce à quoi on détecte la présence de ladite sonde acide nucléique hybridée avec le gène à détecter.
